(19) Europäisches Patentamt
European Patent Office
Office européen des brevets

(11) **EP 4 417 965 A1**

(12) **EUROPEAN PATENT APPLICATION**
published in accordance with Art. 153(4) EPC

(43) Date of publication:
**21.08.2024 Bulletin 2024/34**

(21) Application number: **22881046.1**

(22) Date of filing: **12.10.2022**

(51) International Patent Classification (IPC):
**G01N 21/17** (2006.01)

(52) Cooperative Patent Classification (CPC):
**G01N 21/17**

(86) International application number:
**PCT/JP2022/038043**

(87) International publication number:
**WO 2023/063351 (20.04.2023 Gazette 2023/16)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **15.10.2021 JP 2021169481**

(71) Applicant: **University of Tsukuba
Ibaraki 305-8577 (JP)**

(72) Inventors:
• **YASUNO Yoshiaki
Tsukuba-shi, Ibaraki 305-8577 (JP)**
• **ZHU Lida
Tsukuba-shi, Ibaraki 305-8577 (JP)**
• **MAKITA Shuichi
Tsukuba-shi, Ibaraki 305-8577 (JP)**

(74) Representative: **Mewburn Ellis LLP
Aurora Building
Counterslip
Bristol BS1 6BX (GB)**

(54) **INFORMATION PROCESSING DEVICE, INFORMATION PROCESSING METHOD, AND PROGRAM**

(57) An information processing device includes a reception unit that receives a plurality of OCT images captured under different image-capturing conditions and represented by complex signals, an image processing unit that performs digital refocusing by complex signal processing or digital aberration correction by the complex signal processing on each of the plurality of OCT images received by the reception unit, and a synthesis unit that synthesizes the plurality of OCT images on which the image processing unit has performed the digital refocusing by the complex signal processing or the digital aberration correction by the complex signal processing.

FIG. 1

**Description**

Technical Field

Embodiments of the present invention relate to an information processing device, an information processing method, and a program.

The present application claims priority based on JP 2021-169481 filed in Japan on October 15, 2021, the contents of which are incorporated herein by reference.

Background Art

Optical coherence tomography (OCT) is a technique for acquiring tomographic images of a specimen (mainly a living body) utilizing coherence of light. OCT can acquire images showing not only the surface of a specimen but also the internal structure of the specimen with high spatial resolution.

OCT has been put into practical use for ophthalmic retinal diagnosis. In addition, OCT is used for visualization and quantification of characteristics (for example, statistical properties of directionality and size) of a tissue structure and a microfilament structure below optical resolution while taking, as a specimen, a tissue to be observed such as a cultured tissue, an in vitro (inside a living body) sample, or an ex vivo (outside a living body) sample.

Regarding OCT, there is known a technology for suppressing coherent noise caused by multiply scattered light by randomizing the wave front of probe light (see, for example, NPTL 1). A technology called "OCT microscope (OCM)" for imaging an ex vivo specimen using an optical coherence tomograph (OCT) has been studied.

Citation List

Non-Patent Literature

NPTL 1: DAWID BORYCKI, MICHAL HAMKALO, MACIEJ NOWAKOWSKI, MACIEJ SZKULMOWSKI, AND MACIEJ WOJTKOWSKI "Spatiotemporal optical coherence (STOC) manipulation suppresses coherent cross-talk in full-field swept-source optical coherence tomography", Vol. 10, No. 4 | 1 Apr 2019 | BIOMEDICAL OPTICS EXPRESS 2032

Summary of Invention

Technical Problem

In the technique described in NPTL 1, an optical deformable mirror and a device called a full field OCT are required for implementation. Affinity may be low depending on the OCT. Theoretically, the principle of operation is partially unclear.

The OCT and OCM are utilized to visualize tissue structures at millimeter depths. An image obtained by OCT (OCT image) is reconstructed based on reflected light (backscattered light) from a tissue site.

However, at a tissue site deeper than about 1.5 millimeters, noise caused by multiple scattering of the probe light is dominant with respect to the reflected light from the tissue site. Because of this, at a tissue site deeper than about 1.5 millimeters, noise caused by multiple scattering of the probe light is overlaid on the reflected light from the tissue site, which may obscure the morphology of the specimen when visualized. An object of the present invention is to provide an information processing device, an information processing method, and a program capable of reducing the influence of noise (artifact) caused by multiple scattering of probe light.

Solution to Problem

An embodiment of the present invention is an information processing device including: a reception unit that receives a plurality of OCT images captured under different image-capturing conditions and represented by complex signals; an image processing unit that performs digital refocusing by complex signal processing or digital aberration correction by the complex signal processing on each of the plurality of OCT images received by the reception unit; and a synthesis unit that synthesizes the plurality of OCT images on which the image processing unit has performed the digital refocusing by the complex signal processing or the digital aberration correction by the complex signal processing.

According to an embodiment of the present invention, in the information processing device, each of the plurality of OCT images is captured using an optical system having a different aberration.

According to an embodiment of the present invention, in the information processing device, each of the plurality of OCT images is captured at a different focal position or captured using an optical system having a different defocus aberration.

According to an embodiment of the present invention, in the information processing device, each of the plurality of OCT images is captured at a different focal position obtained by any of changing a distance with respect to a specimen for a measuring beam, the specimen in an incident direction to an objective lens, or the objective lens; utilizing an optical system that changes refractive power; using an electrically controlled tunable lens; utilizing a spatial light modulator; and rotating an optical element having aberration.

According to an embodiment of the present invention, in the information processing device, each of the plurality of OCT images is an image captured by performing at least one of a change in a position of an objective lens, a change in a position of a specimen, or a change in an angle of the specimen with respect to an incident direction of a measuring beam to the objective lens.

According to an embodiment of the present invention, in the information processing device, the image processing unit performs any of forward-model based refocusing, digital adaptive optics, and interferometric synthetic aperture microscopy.

According to an embodiment of the present invention, in the information processing device, the synthesis unit performs complex averaging or intensity and amplitude averaging based on the plurality of OCT images.

According to an embodiment of the present invention, in the information processing device, the synthesis unit corrects a relative phase between the plurality of OCT images and then synthesizes the plurality of OCT images.

An embodiment of the present invention is an information processing method executed by a computer, the method including: receiving a plurality of OCT images captured under different image-capturing conditions and represented by complex signals; performing digital refocusing by complex signal processing or digital aberration correction by the complex signal processing on each of the plurality of OCT images; and synthesizing the plurality of OCT images on which the digital refocusing by the complex signal processing or the digital aberration correction by the complex signal processing has been performed.

An embodiment of the present invention is a computer program for causing a computer to execute a process, the process including: receiving a plurality of OCT images captured under different image-capturing conditions and represented by complex signals; performing digital refocusing by complex signal processing or digital aberration correction by the complex signal processing on each of the plurality of OCT images; and synthesizing the plurality of OCT images on which the digital refocusing by the complex signal processing or the digital aberration correction by the complex signal processing has been performed.

Advantageous Effects of Invention

According to the embodiments of the present invention, it is possible to provide an information processing device, an information processing method, and a program capable of reducing the influence of noise caused by multiple scattering of probe light.

Brief Description of Drawings

FIG. 1 is a diagram illustrating an example of a configuration of an information processing device according to an embodiment of the present invention.
FIG. 2 is a flowchart illustrating an example of operation of the information processing device according to the present embodiment.
FIG. 3 is a diagram illustrating an example of a configuration of an information processing device according to a modification of an embodiment.
FIG. 4 is a flowchart illustrating an example of operation of an information processing device according to a modification of an embodiment.
FIG. 5 is a diagram illustrating Example 1 of a processing result of an information processing device according to a modification of an embodiment.
FIG. 6 is a diagram illustrating Example 2 of a processing result of an information processing device according to a modification of an embodiment.
FIG. 7 is a diagram illustrating Example 3 of a processing result of an information processing device according to a modification of an embodiment.
FIG. 8 is a diagram illustrating Example 4 of a processing result of an information processing device according to a modification of an embodiment.

Description of Embodiments

Next, an information processing device, an information processing method, and a program according to the present embodiment will be described with reference to the accompanying drawings. The embodiment described below is merely an example, and embodiments to which the present invention is applied are not limited to the following embodiment.

In all the drawings for describing the embodiments, components having the same functions are denoted by the same reference signs, and repeated description thereof is omitted.

The expression "based on XX" in the present application means "based on at least XX", and includes a case of being based on another element in addition to XX. Further, the expression "based on XX" is not limited to a case where XX is directly used, and includes a case of being based on an element obtained by subjecting XX to calculation, processing, or the like. "XX" can be any element (for example, optional information).

Embodiment

Information Processing Device

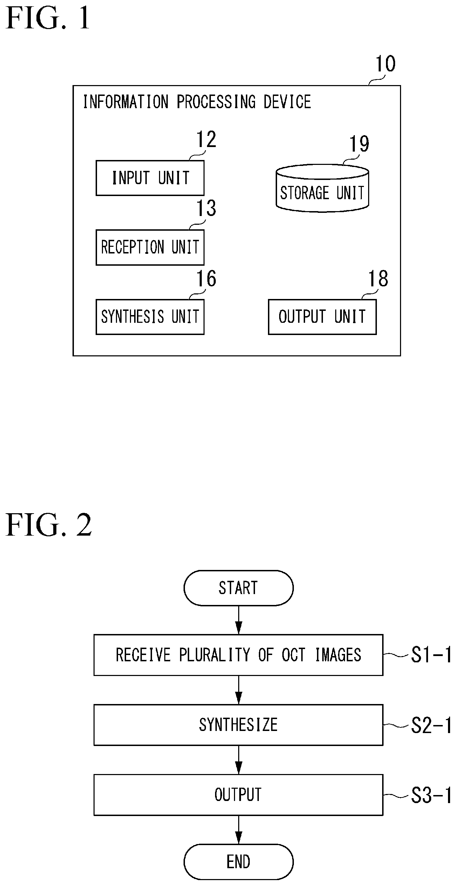

FIG. 1 is a diagram illustrating an example of a configuration of an information processing device according to an embodiment of the present invention. An information processing device 10 according to the present embodiment receives a plurality of OCT images captured under different image-capturing conditions and represented by complex signals. An example of each of the plurality of OCT images is an image of a front surface where a plane to be observed is perpendicular to a depth direction of a specimen. Here, the depth direction of the specimen refers to an incident direction of a measuring beam to the specimen. The front surface is also referred to as an en-face surface.

The information processing device 10 synthesizes the received plurality of OCT images. The information processing device 10 outputs a result obtained by synthesizing the plurality of OCT images.

To reduce the influence of noise (artifact) caused by multiple scattering of probe light, the information processing device 10 acquires the plurality of OCT images captured under different image-capturing conditions such as focal positions, and performs complex averaging on each of the acquired plurality of OCT images.

For example, the focal position is shifted by translating the sample (specimen) in an optical axis direction. The optical axis direction is an incident direction of the measuring beam to the specimen. By performing image capture every time the focal position is shifted, the acquired plurality of OCT images undergo complex averaging. As a result, noise caused by multiple scattering between the plurality of OCT images can be decorrelated, and thus the influence of noise can be reduced.

The information processing device 10 is achieved by an apparatus such as a personal computer, a server, a smartphone, a tablet computer, or an industrial computer. The information processing device 10 includes, for example, an input unit 12, a reception unit 13, a synthesis unit 16, an output unit 18, and a storage unit 19.

The input unit 12 is input with information. As an example, the input unit 12 may include an operation unit such as a keyboard and a mouse. In this case, the input unit 12 is input with information corresponding to operation performed on the operation unit by a user. As another example, the input unit 12 may be input with information from an external device. The external device may be, for example, a portable storage medium. A plurality of OCT images are input to the input unit 12. An example of each of the plurality of OCT images is an image where a plane to be observed is a front surface (en-face surface) perpendicular to the depth direction of the specimen. Each of the plurality of OCT images is captured under a different image-capturing condition and is represented by a complex signal. An example of the image-capturing condition is a focal position, and each of the plurality of OCT images is captured at a different focal position. Specifically, an example of each of the plurality of OCT images is an image captured at a different focal position obtained by changing a distance (a position in the depth direction) with respect to the specimen for the measuring beam, to the specimen in the incident direction to the objective lens, or to the objective lens. For example, the focal position is shifted by translating the specimen in the optical axis direction.

An example of the object to be observed as a specimen is a human or animal living organism, or a non-biological object. Examples of the living organism include a fundus, blood vessel, tooth, and subcutaneous tissue. Examples of the non-biological object include artificial structures such as an electronic component and a mechanical component, natural structures such as a stone and a mineral, and substances that do not have any specific shape.

The reception unit 13 receives a plurality of OCT images input to the input unit 12. Each of the plurality of OCT images is represented by a complex signal. The reception unit 13 stores each of the received plurality of OCT images in the storage unit 19.

The synthesis unit 16 acquires the plurality of OCT images stored in the storage unit 19. The synthesis unit 16 synthesizes the acquired plurality of OCT images. Specifically, the synthesis unit 16 performs synthesizing by averaging (complex-averaging) the plurality of OCT images represented by complex signals.

A multiply scattered signal ~Sj(x, y, z), light illumination, and a scattering path vary depending on a focus configuration. Here, j indicates the j-th focus configuration. Because of this, by averaging each of the plurality of OCT images represented by complex signals, a noise pattern formed by multiply scattered signals is made decorrelated among those collected at different focal positions, and averaged. Thus, the influence of noise caused by multiple scattering of the probe light is reduced or eliminated.

The output unit 18 outputs information. As an example, the output unit 18 may include a display having a screen. In this case, the output unit 18 displays information on the screen. As another example, the output unit 18 may output information to an external device. The external device may be, for example, a portable storage medium.

The output unit 18 acquires a result of synthesizing each of the plurality of OCT images from the synthesis unit 16 and outputs the acquired result of synthesizing each of the plurality of OCT images.

The storage unit 19 stores information. The storage unit 19 stores the plurality of OCT images represented by complex signals output by the reception unit 13.

The reception unit 13, the synthesis unit 16, and the output unit 18 are achieved by a hardware processor such as a central processing unit (CPU) executing a computer program (software) stored in the storage unit 19, for example.

Some or all of these function units may be implemented by hardware (including circuitry) such as a large scale integration (LSI), an application specific integrated circuit (ASIC), a field-programmable gate array (FPGA), and a graphics processing unit (GPU), or may be implemented by cooperation between software and hardware.

The computer program may be stored in advance in a storage device such as a hard disk drive (HDD) or a flash memory, or it may be stored in an attachable and detachable storage medium such as a digital versatile disc (DVD) or a CD-ROM and may be installed when the storage medium is mounted on a drive device.

Operation of Information Processing Device 10

FIG. 2 is a flowchart illustrating an example of operation of the information processing device according to the present embodiment. With reference to FIG. 2, the operation performed after a plurality of images captured under different image-capturing conditions are input to the information processing device 10 will be described.

Step S 1-1

The reception unit 13 receives a plurality of OCT images input to the input unit 12. The reception unit 13 stores each of the received plurality of OCT images in the storage unit 19.

Step S2-1

The synthesis unit 16 acquires each of the plurality of OCT images from the storage unit 19. Each of the plurality of OCT images is represented by a complex signal. The synthesis unit 16 averages each of the acquired plurality of OCT images to synthesize the OCT images.

Step S3-1

The output unit 18 acquires a result of synthesizing each of the plurality of OCT images from the synthesis unit 16 and outputs the acquired result of synthesizing each of the plurality of OCT images.

The case where the plane to be observed is a front surface perpendicular to the depth direction of the specimen has been described in the embodiment mentioned above, but the present invention is not limited thereto. For example, the present invention can be applied to a case where the plane to be observed is a plane crossing a part of or the whole of a region to be observed.

In the embodiment mentioned above, as an example of the different image-capturing condition of each of the plurality of OCT images received by the reception unit 13 in the information processing device 10, the case where image capture is performed at different focal positions has been described, but the present invention is not limited thereto.

For example, as an example of the different image-capturing condition, image capture may be performed by different optical systems. Specifically, each of the plurality of OCT images may be captured using an optical system having a different aberration. In this case, image capture is performed with a different aberration every time image capture is performed. With such a configuration, a plurality of images captured under different image-capturing conditions can be acquired. Further, when optical systems having different aberrations are used, each of the plurality of OCT images may be captured at a different focal position or may be captured using an optical system having a different defocus aberration. In this case, image capture is performed with a different aberration every time image capture is performed. With such a configuration, a plurality of images captured under different image-capturing conditions can be acquired.

In the embodiment mentioned above, the case as follows has been described: in the information processing device 10, each of the plurality of OCT images to be received by the reception unit 13 is an image captured at a different focal position obtained by changing a distance (a position in the depth direction) with respect to the specimen for the measuring beam, to the specimen in the incident direction to the objective lens, or to the objective lens, but the present invention is not limited thereto.

For example, the focal position may be shifted by using an optical system that changes refractive power. In this case, image capture is performed every time the refractive power is changed to shift the focal position. With such a configuration, a plurality of images captured under different image-capturing conditions can be acquired. Further, for example, the focal position may be shifted by using a tunable lens. In this case, image capture is performed every time the focal position is shifted using the tunable lens. With such a configuration, a plurality of images captured under different image-capturing conditions can be acquired.

In addition, for example, each of the plurality of OCT images may be an image captured by performing at least one of a change in a position of the objective lens, a change in a position of the specimen, or a change in an angle (tilt angle) of the specimen with respect to the incident direction of the measuring beam to the objective lens. With such a configuration, a plurality of images captured under different image-capturing conditions can be acquired.

Further, for example, the focal position may be shifted by using a spatial light modulator. In this case, image capture is performed every time the focal position is shifted using the spatial light modulator. With such a configuration, a plurality of images captured under different image-capturing conditions can be acquired. Further, for example, the focal position may be shifted by rotating an optical element having aberration. In this case, image capture is performed every time the focal position is shifted by rotating the optical element having aberration. With such a configuration, a plurality of images captured under different image-capturing conditions can be acquired.

In the embodiment mentioned above, the case where, in the information processing device 10, the synthesis unit 16 synthesizes the plurality of OCT images by averaging (complex-averaging) the plurality of OCT images represented by complex signals has been described, but the present invention is not limited thereto.

For example, the synthesis unit 16 may synthesize the OCT images by averaging (intensity-averaging) the intensity of each of the plurality of OCT images represented by complex signals. By averaging the intensity of each of the plurality of OCT images represented by complex signals, a noise pattern formed by multiply scattered signals is made decorrelated among those collected at different focal positions, and averaged. Thus, the influence of noise caused by multiple scattering of the probe light is reduced or eliminated.

For example, the synthesis unit 16 may synthesize the OCT images by averaging (amplitude-averaging) the amplitude (complex amplitude) of the plurality of OCT images represented by complex signals. By averaging the amplitude of the plurality of OCT images represented by complex signals, a noise pattern formed by multiply scattered signals is made decorrelated among those collected at different focal positions, and averaged. Thus, the influence of noise caused by multiple scattering of the probe light is reduced or eliminated.

In the above-described embodiment, the synthesis unit 16 may correct a relative phase between the plurality of OCT images acquired from the storage unit 19 and then synthesize the plurality of OCT images having undergone the relative phase correction. With such a configuration, since the relative phase between the plurality of OCT images can be corrected, the contrast of the image obtained by synthesizing each of the plurality of OCT images having undergone the relative phase correction can be improved.

In the case where the relative phase between the plurality of OCT images is corrected, the synthesis unit 16 may synthesize the plurality of OCT images by averaging (complex-averaging) the plurality of OCT images having undergone the relative phase correction, averaging (intensity-averaging) the intensities of the plurality of OCT images having undergone the relative phase correction, or averaging (amplitude-averaging) the amplitude (complex amplitude) of the plurality of OCT images having undergone the relative phase correction.

According to the information processing device 10 of the present embodiment, the information processing device 10 includes the reception unit 13 that receives a plurality of OCT images captured under different image-capturing conditions and represented by complex signals, and the synthesis unit 16 that synthesizes the plurality of OCT images received by the reception unit 13.

With such a configuration, the information processing device 10 can synthesize a plurality of OCT images captured under different image-capturing conditions. Thus, since the multiply scattered light is reduced or removed by being averaged, singly scattered light remains, and the OCT image is reconstructed based on the singly scattered light. Because of this, the influence of noise caused by the multiply scattered light of the probe light can be reduced or eliminated, and therefore the morphology of the specimen becomes clear when visualized, as compared to a case where the plurality of OCT images are not synthesized. This makes it possible to expand the depth measurement range, and thus the use value and application range of the OCT and OCM can be expanded. For example, in fundus diagnosis, lesions of choroid and sclera can be visualized, and types of diseases that can be diagnosed by OCT can be significantly expanded. In addition, with the OCM, it is possible to measure the entire depth of an organoid, and the application range of the OCM can be largely expanded.

In the information processing device 10, each of the plurality of OCT images is captured at a different focal position or captured using an optical system having a different aberration.

With such a configuration, image capture can be performed with a different focal position or with a different aberration every time image capture is performed. Therefore, a plurality of OCT images captured under different image-capturing conditions can be acquired.

In the information processing device 10, each of the plurality of OCT images is captured at a different focal position or captured using an optical system having a different defocus aberration.

With such a configuration, image capture can be performed by changing a focal position for each image capture or by using an optical system having a different defocus aberration for each image capture. Therefore, a plurality of OCT images captured under different image-capturing conditions can be acquired.

In the information processing device 10, each of the plurality of OCT images is captured at a different focal position obtained by any of changing a distance (a position in the depth direction) with respect to a specimen for a measuring beam, to the specimen in an incident direction to an objective lens, or to the objective lens; utilizing an optical system that changes refractive power; using an electrically controlled tunable lens; utilizing a spatial light modulator; and rotating an optical element having aberration. With such a configuration, it is possible to acquire a plurality of OCT images captured at different focal positions. This is because image capturing can be performed with a focal position being changed for each image capture by any of changing a distance (a position in the depth direction) with respect to a specimen for the measuring beam, to the specimen in the incident direction to the objective lens, or to the objective lens; utilizing an optical system that changes refractive power; using an electrically controlled tunable lens; utilizing a spatial light modulator; and rotating an optical element having aberration.

In the information processing device 10, each of the plurality of OCT images is an image captured by performing at least one of a change in a position of the objective lens, a change in a position of the specimen, or a change in an angle of the specimen with respect to the incident direction of the measuring beam to the objective lens.

With such a configuration, each of the plurality of OCT images is captured by performing at least one of a change in a position of the objective lens, a change in a position of the specimen, or a change in an angle of the specimen with respect to the incident direction of the measuring beam to the objective lens, and therefore the plurality of OCT images captured at different focal positions can be acquired.

In the information processing device 10, the synthesis unit 16 performs complex averaging or intensity and amplitude averaging based on the plurality of OCT images. With such a configuration, the synthesis unit 16 can synthesize a plurality of OCT images represented by complex signals by performing complex averaging or intensity and amplitude averaging on each of the plurality of OCT images. Multiple scattering can be reduced or removed by performing complex averaging or intensity and amplitude averaging on each of the plurality of OCT images represented by complex signals, thereby making it possible to reduce or eliminate the influence of noise caused by the multiple scattering of the probe light.

In the information processing device 10, the synthesis unit 16 corrects a relative phase between the plurality of OCT images and then synthesizes the plurality of OCT images. With such a configuration, since the relative phase between the plurality of OCT images can be corrected, the contrast of the image obtained by synthesizing each of the plurality of OCT images having undergone the relative phase correction can be improved.

Modification of Embodiment

Information Processing Device

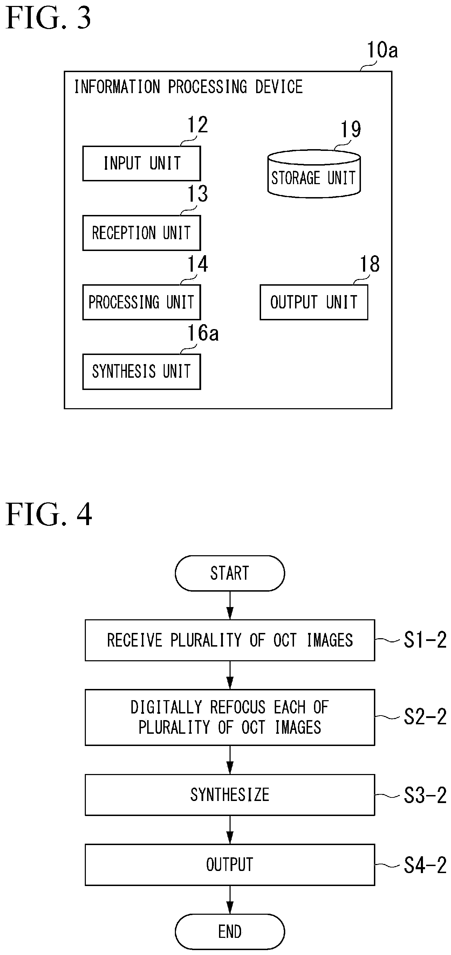

FIG. 3 is a diagram illustrating an example of a configuration of an information processing device according to a modification of the embodiment. An information processing device 10a according to the modification of the embodiment receives a plurality of OCT images captured under different image-capturing conditions and represented by complex signals. An example of each of the plurality of OCT images is an image where a plane to be observed is a front surface (en-face surface) perpendicular to the depth direction of a specimen.

The information processing device 10a performs digital refocusing on each of the received plurality of OCT images. Digital refocusing is focusing using digital processing in a frequency space. To be specific, the information processing device 10a performs digital refocusing on each of the received plurality of OCT images by complex signal processing. The information processing device 10a synthesizes the plurality of OCT images having undergone the digital refocusing. The information processing device 10a outputs a result obtained by synthesizing the plurality of OCT images having undergone the digital refocusing.

The information processing device 10a uses a multi-focus complex fusion (MFCF) method to reduce the influence of noise caused by multiple scattering of probe light. In the MFCF method, a plurality of OCT images captured under different image-capturing conditions such as focal positions are acquired, each of the acquired plurality of OCT images is refocused by calculation, and the refocused plurality of OCT images undergo complex averaging.

For example, the focal position is shifted by translating the sample (specimen) in an optical axis direction. Each of the plurality of OCT images acquired by performing image capture every time the focal position is shifted is at a different focal position, and therefore the image itself is different from another. Then, an in-focus image is produced by performing digital refocusing on each of the plurality of OCT images. As a result, the plurality of OCT images all have the same appearance. However, noise caused by multiple scattering of the probe light is overlaid on each of the plurality of OCT images having undergone refocusing.

Since the noise caused by multiple scattering between the plurality of OCT images can be decorrelated by complex-averaging the refocused plurality of OCT images, the information processing device 10a can reduce the influence of the noise.

The information processing device 10a is achieved by an apparatus such as a personal computer, a server, a smartphone, a tablet computer, or an industrial computer. The information processing device 10 includes, for example, an input unit 12, a reception unit 13, a processing unit 14, a synthesis unit 16a, an output unit 18, and a storage unit 19.

The processing unit 14 acquires a plurality of OCT images stored in the storage unit 19. The processing unit 14 performs various kinds of processing on each of the acquired plurality of OCT images. The processing unit 14 performs (digital) refocusing processing by calculation on each of the acquired plurality of OCT images. The processing unit 14 performs (digital) refocusing processing by complex signal processing on each of the plurality of OCT images. A plane to be observed is represented by "(x, y)", and a position in the depth direction from a surface to be observed is represented by "z". In a case of a single backscattered signal S(x, y, z) on a sample surface (surface to be observed) at a specific distance Zd from the focal plane, the processing unit 14 corrects a two-dimensional spatial frequency filter $H^{-1}(f_x, f_y; z_d)$ based on Formula (1).

[Math. 1]

$$H^{-1}(f_x, f_y;\ z_d) = \exp\left[-i\pi\lambda_c z_d(f_x{}^2 + f_y{}^2)\right],\quad \cdots (1)$$

In Formula (1), fx and fy are spatial frequencies corresponding to positions in the lateral direction of x and y, and λc is a center wavelength. A signal S'(x, y, z) with the defocus corrected can be expressed as Formula (2).

[Math. 2]

$$S'(x, y, z) = \mathcal{F}^{-1}\{\mathcal{F}[S(x, y, z)]H^{-1}(f_x, f_y;\ z_d)\},\quad \cdots (2)$$

In Formula (2), F represents a two-dimensional Fourier transform at a front surface to be observed, and $F^{-1}$ represents a two-dimensional inverse Fourier transform. Even when the distance between the sample surface and the focal plane is changed to Z'd by shifting the focal position, the defocus can be corrected by using a corresponding two-dimensional spatial frequency filter $H^{-1}(f_x, f_y; Z'd)$. Accordingly, the refocused backscattered signal S'(x, y, z) converges on the same complex signal even when the focus configurations are different.

The synthesis unit 16a acquires each of the plurality of OCT images having undergone the refocusing processing from the processing unit 14. The synthesis unit 16a synthesizes the acquired plurality of OCT images having undergone the refocusing processing. Specifically, the synthesis unit 16 averages (complex-averaging) the acquired plurality of OCT images having undergone the refocusing processing and represented by complex signals to synthesize the OCT images.

A multiply scattered signal ~Sj(x, y, z), light illumination, and a scattering path vary depending on a focus configuration. Here, j indicates the j-th focus configuration. By averaging each of the plurality of OCT images having undergone the refocusing processing and represented by complex signals, a noise pattern formed by multiply scattered signals is made decorrelated among those collected at different focal positions, and averaged. Thus, the influence of noise caused by multiple scattering of the probe light is reduced or eliminated.

The output unit 18 outputs information. As an example, the output unit 18 may include a display having a screen. In this case, the output unit 18 displays information on the screen. As another example, the output unit 18 may output information to an external device. The external device may be, for example, a portable storage medium.

The output unit 18 acquires a result of synthesizing the plurality of OCT images having undergone the refocusing processing from the synthesis unit 16a, and outputs the acquired result of synthesizing the plurality of OCT images having undergone the refocusing processing.

The storage unit 19 stores information. The storage unit 19 stores the plurality of OCT images represented by complex signals output by the reception unit 13.

The reception unit 13, the processing unit 14, the synthesis unit 16a, and the output unit 18 are achieved by a hardware processor such as a CPU executing a computer program stored in the storage unit 19, for example.

Some or all of these function units may be implemented by hardware (including circuitry) such as an LSI, an ASIC, an FPGA, and a GPU, or may be implemented by cooperation between software and hardware.

The computer program may be stored in advance in a storage device such as an HDD or a flash memory, or it may be stored in an attachable and detachable storage medium such as a DVD or a CD-ROM and may be installed when the storage medium is mounted on a drive device.

Operation of Information Processing Device 10a

FIG. 4 is a flowchart illustrating an example of operation of the information processing device according to the modification of the embodiment. With reference to FIG. 4, operation performed after a plurality of images captured under different image-capturing conditions are input to the information processing device 10a will be described.

Step S1-2

The reception unit 13 receives a plurality of OCT images input to the input unit 12. The reception unit 13 stores the received plurality of OCT images in the storage unit 19.

Step S2-2

The processing unit 14 acquires the plurality of OCT images stored in the storage unit 19. The processing unit 14 performs (digital) refocusing processing by calculation on each of the acquired plurality of OCT images. For example, the processing unit 14 performs (digital) refocusing processing by complex signal processing on each of the plurality of OCT images.

Step S3-2

The synthesis unit 16a acquires each of the plurality of OCT images having undergone the refocusing processing from the processing unit 14. The synthesis unit 16a averages each of the acquired plurality of OCT images having undergone the refocusing processing to synthesize the OCT images.

Step S4-2

The output unit 18 acquires a result of synthesizing each of the plurality of OCT images having undergone the refocusing processing from the synthesis unit 16a, and outputs the acquired result of synthesizing each of the plurality of OCT images having undergone the refocusing processing.

The case where the plane to be observed is a front surface perpendicular to the depth direction of the specimen has been described in the embodiment mentioned above, but the present invention is not limited thereto. For example, the present invention can be applied to a plane where the plane to be observed crosses a part of or the whole of a region to be observed.

In the embodiment mentioned above, as an example of the different image-capturing condition of each of the plurality of OCT images received by the reception unit 13 in the information processing device 10a, the case where image capture is performed at different focal positions has been described, but the present invention is not limited thereto.

For example, as an example of the different image-capturing condition, image capture may be performed by different optical systems. Specifically, each of the plurality of OCT images may be captured using an optical system having a different aberration. In this case, image capture is performed with a different aberration every time image capture is performed. With such a configuration, a plurality of images captured under different image-capturing conditions can be acquired. Further, when optical systems having different aberrations are used, each of the plurality of OCT images may be captured at a different focal position or may be captured using an optical system having a different defocus aberration. In this case, image capture is performed with a different aberration every time image capture is performed. With such a configuration, a plurality of images captured under different image-capturing conditions can be acquired.

In the embodiment mentioned above, the case as follows has been described: in the information processing device 10, each of the plurality of OCT images to be received by the reception unit 13 is an image captured at a different focal position obtained by changing a distance (a position in the depth direction) with respect to the specimen for the measuring beam, to the specimen in the incident direction to the objective lens, or to the objective lens, but the present invention is not limited thereto.

For example, the focal position may be shifted by using an optical system that changes refractive power. In this case, image capture is performed every time the refractive power is changed to shift the focal position. With such a configuration, a plurality of images captured under different image-capturing conditions can be acquired. Further, for example, the focal position may be shifted by using a tunable lens. In this case, image capture is performed every time the focal position is shifted using the tunable lens. With such a configuration, a plurality of images captured under different image-capturing conditions can be acquired.

In addition, for example, each of the plurality of OCT images may be an image captured by performing at least one of a change in a position of the objective lens, a change in a position of the specimen, or a change in an angle (tilt angle) of the specimen with respect to the incident direction of the measuring beam to the objective lens. With such a configuration, a plurality of images captured under different image-capturing conditions can be acquired.

Further, for example, the focal position may be shifted by using a spatial light modulator. In this case, image capture is performed every time the focal position is shifted using the spatial light modulator. With such a configuration, a plurality of images captured under different image-capturing conditions can be acquired. Further, for example, the focal position may be shifted by rotating an optical element having aberration. In this case, image capture is performed every time the focal position is shifted by rotating the optical element having aberration. With such a configuration, a plurality of images captured under different image-capturing conditions can be acquired.

In the above-described embodiment, the case where the processing unit 14 performs digital refocusing on each of the plurality of OCT images in the information processing device 10a has been described, but the present invention is not limited thereto.

For example, the processing unit 14 may perform digital aberration correction on each of the plurality of OCT images. Specifically, the processing unit 14 performs digital aberration correction by complex signal processing on each of the plurality of OCT images. In this case, the synthesis unit 16a acquires each of the plurality of OCT images having undergone digital aberration correction from the processing unit 14. The synthesis unit 16a synthesizes the acquired plurality of OCT images having undergone the digital aberration correction. Specifically, the synthesis unit 16a averages (complex-averaging) the acquired plurality of OCT images having undergone the digital aberration correction and represented by complex signals to synthesize the OCT images. When performing the digital aberration correction, the processing unit 14 may perform any of digital adaptive optics and interferometric synthetic aperture microscopy. In this case, the synthesis unit 16a acquires, from the processing unit 14, each of the plurality of OCT images having undergone the digital aberration correction by any of the digital adaptive optics and the interferometric synthetic aperture microscopy (ISAM). The synthesis unit 16a synthesizes each of the acquired plurality of OCT images having undergone the digital aberration correction.

In the above-described embodiment, the digital refocusing may be performed by forward-model based refocusing. In this case, the synthesis unit 16a acquires each of the plurality of OCT images having undergone the digital refocusing by the forward-model based refocusing from the processing unit 14. The synthesis unit 16a synthesizes the acquired plurality of OCT images having undergone the digital aberration correction.

In the above-described embodiment, the case where the synthesis unit 16a averages (complex-averaging) the plurality of OCT images represented by complex signals to synthesize the OCT images in the information processing device 10a has been described, but the present invention is not limited thereto.

For example, the synthesis unit 16a may average (intensity-averaging) the intensities of the plurality of OCT images represented by complex signals to synthesize the OCT images. By averaging the intensities of the plurality of OCT images represented by complex signals, a noise pattern formed by multiply scattered signals is made decorrelated among those collected at different focal positions, and averaged. Thus, the influence of noise caused by multiple scattering of the probe light is reduced or eliminated.

For example, the synthesis unit 16a may average (amplitude-averaging) the amplitude (complex amplitude) of the plurality of OCT images represented by complex signals to synthesize the OCT images. By averaging the amplitude of the plurality of OCT images represented by complex signals, a noise pattern formed by multiply scattered signals is made decorrelated among those collected at different focal positions, and averaged. Thus, the influence of noise caused by multiple scattering of the probe light is reduced or eliminated.

In the above-described embodiment, the synthesis unit 16a may correct a relative phase between the plurality of OCT images having undergone the refocusing processing, which are acquired from the processing unit 14, and then may synthesize the plurality of OCT images having undergone the refocusing processing and the relative phase correction.

With such a configuration, since the relative phase between the plurality of OCT images having undergone the refocusing processing can be corrected, the contrast of the image obtained by synthesizing the plurality of OCT images having undergone the refocusing processing and the relative phase correction can be improved.

In this case, the synthesis unit 16a may average (complex-averaging) the plurality of OCT images having undergone the refocusing processing and the relative phase correction to synthesize the OCT images, may average (intensity-averaging) the intensities of the plurality of OCT images having undergone the refocusing processing and the relative phase correction to synthesize the OCT images, or may average (amplitude-averaging) the amplitude (complex amplitude) of the plurality of OCT images having undergone the refocusing processing and the relative phase correction to synthesize the OCT images.

According to the information processing device 10a in the modification of the embodiment, the information processing device 10a includes: the reception unit 13 that receives a plurality of OCT images captured under different image-capturing conditions and represented by complex signals; an image processing unit as the processing unit 14 that performs digital refocusing by complex signal processing or digital aberration correction by the complex signal processing on each of the plurality of OCT images received by the reception unit 13; and the synthesis unit 16a that synthesizes the plurality of OCT images on which the image processing unit has performed the digital refocusing by the complex signal processing or the digital aberration correction by the complex signal processing.

With such a configuration, the information processing device 10a can acquire a plurality of in-focus OCT images based on each of the plurality of OCT images captured under different image-capturing conditions. The information processing device 10a can synthesize the plurality of in-focus OCT images. Thus, since multiply scattered light is removed by being averaged, singly scattered light remains, and the OCT image is reconstructed based on the singly scattered light. Because of this, the influence of noise caused by the multiple scattering of the probe light can be reduced, and therefore the morphology of the specimen becomes clear when visualized, as compared to a case where the plurality of OCT images are not synthesized. Furthermore, as compared to a case where digital refocusing by complex signal processing or digital aberration correction by complex signal processing is not performed, a plurality of in-focus OCT images can be synthesized, and thus the morphology of the specimen becomes clearer when visualized. This makes it possible to expand the depth measurement range, and thus the use value and application range of the OCT and OCM can be expanded. For example, in fundus diagnosis, lesions of choroid and sclera can be visualized, and types of diseases that can be diagnosed by OCT can be significantly expanded. In addition, with the OCM, it is possible to measure the entire depth of an organoid, and the application range of the OCM can be largely expanded.

In the information processing device 10a, each of the plurality of OCT images is captured at a different focal position or captured using an optical system having a different aberration.

With such a configuration, image capture can be performed with a different focal position or with a different aberration every time image capture is performed. Therefore, a plurality of OCT images captured under different image-capturing conditions can be acquired.

In the information processing device 10a, each of the plurality of OCT images is captured at a different focal position or captured using an optical system having a different defocus aberration.

With such a configuration, image capture can be performed by changing a focal position for each image capture or by using an optical system having a different defocus aberration for each image capture. Therefore, a plurality of OCT images captured under different image-capturing conditions can be acquired.

In the information processing device 10a, each of the plurality of OCT images is captured at a different focal position obtained by any of changing a distance (a position in the depth direction) with respect to a specimen for the measuring beam, to the specimen in the incident direction to the objective lens, or to the objective lens; utilizing an optical system that changes refractive power; using an electrically controlled tunable lens; utilizing a spatial light modulator; and rotating an optical element having aberration.

With such a configuration, it is possible to acquire a plurality of OCT images captured at different focal positions. This is because image capturing can be performed with a focal position being changed for each image capture by any of changing a distance (a position in the depth direction) with respect to a specimen for the measuring beam, to the specimen in the incident direction to the objective lens, or to the objective lens; utilizing an optical system that changes refractive power; using an electrically controlled tunable lens; utilizing a spatial light modulator; and rotating an optical element having aberration.

In the information processing device 10a, each of the plurality of OCT images is an image captured by performing at least one of a change in a position of the objective lens, a change in a position of the specimen, or a change in an angle of the specimen with respect to the incident direction of the measuring beam to the objective lens.

With such a configuration, each of the plurality of OCT images is captured by performing at least one of a change in a position of the objective lens, a change in a position of the specimen, or a change in an angle of the specimen with respect to the incident direction of the measuring beam to the objective lens, and therefore the plurality of OCT images captured at different focal positions can be acquired.

In the information processing device 10a, the image processing unit performs any of forward-model based refocusing, digital adaptive optics, and interferometric synthetic aperture microscopy.

With such a configuration, the information processing device 10a can acquire a digitally refocused image by forward-model based refocusing, and can acquire an in-focus image having undergone digital aberration correction by any of digital adaptive optics and interferometric synthetic aperture microscopy.

In the information processing device 10a, the synthesis unit 16a performs complex averaging or intensity and amplitude averaging based on the plurality of OCT images. With such a configuration, the synthesis unit 16a performs complex averaging or intensity and amplitude averaging on each of the plurality of OCT images having undergone the digital refocusing or digital aberration correction, thereby making it possible to synthesize the OCT images. Multiple scattering can be removed by performing the complex averaging or intensity and amplitude averaging on each of the plurality of OCT images having undergone the digital refocusing or digital aberration correction, which makes it possible to reduce or eliminate the influence of noise caused by the multiple scattering of the probe light.

In the information processing device 10a, the synthesis unit 16a corrects a relative phase between the plurality of OCT images having undergone the digital refocusing or digital aberration correction and then synthesizes the plurality of OCT images.

With such a configuration, since the relative phase between the plurality of OCT images having undergone the digital refocusing or digital aberration correction can be corrected, the contrast of the image obtained by synthesizing each of the plurality of OCT images having undergone the relative phase correction can be improved.

An example of an advantageous effect of the information processing device 10a according to the modification of the embodiment will be described below.

First, an OCT system used in the modification will be described. In the OCT system, the center wavelength of the sweep light source is 1.3 $\mu$m and the scanning speed is 50 kHz. The effective focal distance of the used objective lens is 36 mm. The effective NA of the system is 0.048.

The DOF of the OCT system is 0.36 mm in air. The resolution in the lateral direction is 19 $\mu$m and the resolution in the axial direction is 14 $\mu$m in a tissue. The pixel interval along the depth is 7.24 $\mu$m in a tissue. The total acquisition time for a single volume is 6.5 seconds.

Subsequently, a sample and a protocol will be described. The sample used is made of two pieces of cover glass having a thickness of 0.12 mm and a phantom layer having a thickness of 2 mm. The phantom is a mixture of 0.5 mL jelly and 0.03 mL of microparticles at a concentration of 10%. The diameter of the microparticle is 10 $\mu$m. The cover glass on the upper side is slightly tilted to prevent mirror reflection, and the other cover glass on the lower side is used for signal-to-noise ratio (SNR) analysis because there is no reflected signal inside the glass.

For the measurement, a lateral scanning range was 1.5 $\times$ 1.5 mm$^2$, and sampling was performed at 512 $\times$ 512 points. The focal point was manually set to a position lower than a surface of the sample by approximately 2 mm to enhance light collection efficiency in the deep part. Data of two groups was measured. One group, termed a "non-focus-shift" group, was a group for reference, and was acquired by performing six consecutive acquisitions with no focus shift. Thereafter, in the other group, i.e., an MFCF group, the sample was manually moved approximately 0.1 mm along the depth direction in six-time collections, whereby the focal point was manually shifted. The MFCF group corresponds to the modification of the present embodiment. At the same time, the standard delay was adjusted to compensate for a difference in an optical path changed by the focus shift of the sample arm to prevent sensitivity roll-off.

The data processing will be described below. In the MFCF group, the initial refocusing was applied individually to a volume of each of the acquired OCT images represented by complex signals.

Subsequently, one volume was selected as a reference volume, and other volumes were positioned to the reference volume by autocorrelation of signal intensity. Subsequently, a signal from a surface of the lower-side glass under the phantom was used to correct a mutual phase difference.

Finally, six volumes were complex-averaged to produce an averaged volume and the like. In the "non-focus-shift" group as well, the volume production was carried out by performing similar processing. However, focus shifting was not performed.

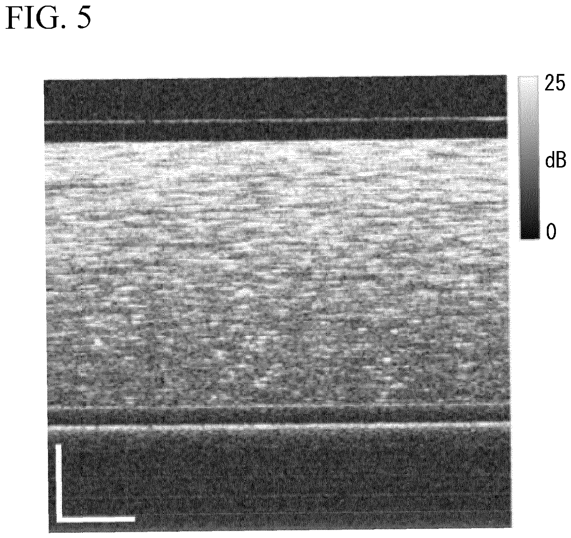

FIG. 5 is a diagram illustrating Example 1 of a processing result of the information processing device according to the modification of the embodiment. FIG. 5 depicts a result of a logarithm intensity B-scan of the sample. In FIG. 5, the scale bar indicates 250 $\mu$m. The sample is from one acquisition volume.

According to FIG. 5, two transparent layers of the cover glass and a thick layer of a phantom can be observed. According to FIG. 5, microparticles in the vicinity of the depth of field in a deep region can be visualized, and strong blurring due to defocus is observed at a shallow depth.

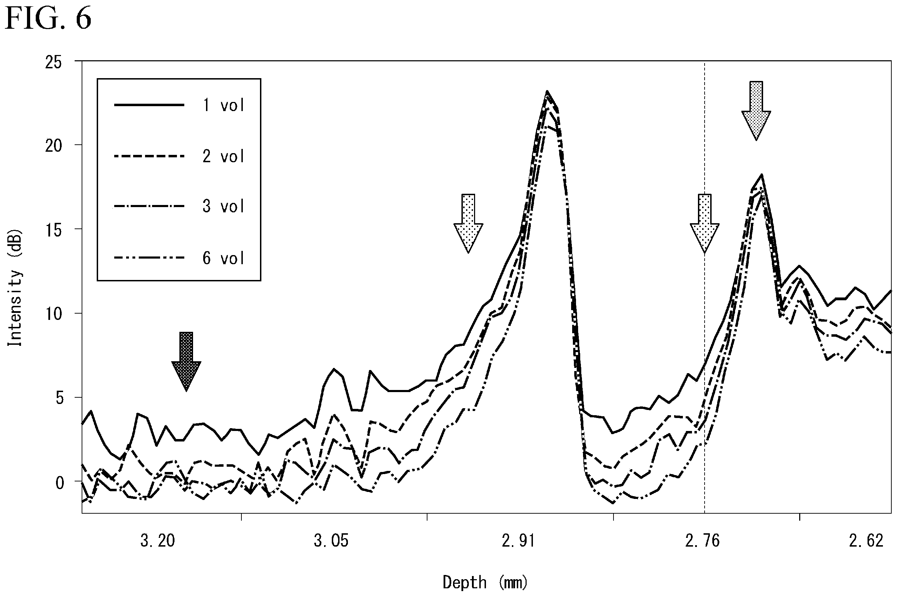

FIG. 6 is a diagram illustrating Example 2 of a processing result of the information processing device according to the modification of the embodiment. FIG. 6 depicts depth (mm)-intensity (dB) profiles of volumes having undergone MFCF processing in the periphery of the lower-side cover glass. The profiles are each an intensity average of 100 adjacent lines. Each profile corresponds to averaging of a different number.

It is understood that the intensity on the upper surface of the glass does not noticeably change compared to the noise signal at deeper depths, as indicated by the rightmost arrow. This indicates that the mutual phase correction works well. A slight change in intensity may be caused by intensity attenuation due to defocus as the focus is shifted. Furthermore, the glass surface signal has a strong asymmetric side lobe in a direction toward a deeper side. This is caused by multiply scattered light.

A very deep region (e.g., about 3.2 mm, the leftmost arrow) indicates a noise floor. This noise floor is reduced by complex averaging, as expected. In a region immediately under the glass surface (around the second and third arrows from the left), the intensity is much higher than that in the noise floor but is suppressed by the averaging. This indicates that the light caused by multiple scattering is reduced or removed. The ratios of the surface signal to the signals at a depth indicated by a broken line are 11.2, 12.2, 13.6, and 14.7 dB for the averages of one, two, three, and six volumes, respectively.

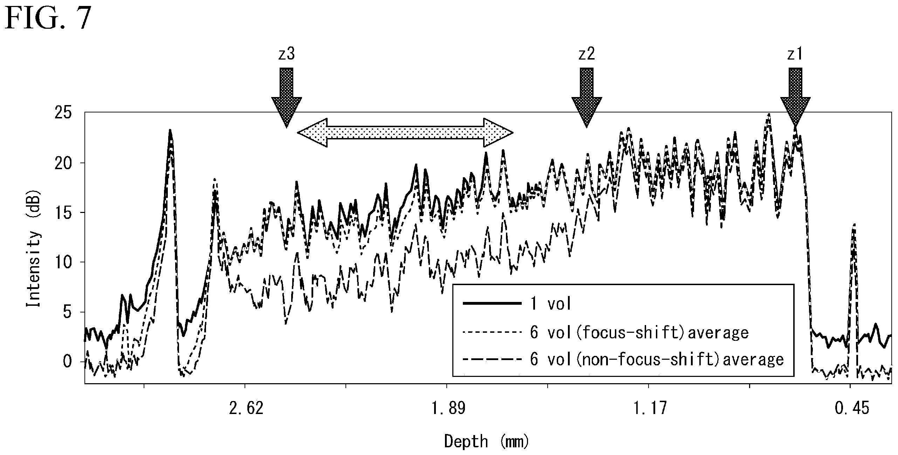

FIG. 7 is a diagram illustrating Example 3 of a processing result of the information processing device according to the modification of the embodiment. In FIG. 7, the horizontal axis represents depth (mm), and the vertical axis represents intensity (dB). The MFCF method and the non-focus-shift method will be described with reference to FIG. 7. FIG. 7 depicts intensity profiles of single volume, non-focus-shift, and MFCF. Each profile is an intensity average of 100 adjacent lines.

According to FIG. 7, the profiles are biased to have approximately the same signal intensity around the first glass surfaces (arrow z1). It is understood that the MFCF further suppresses the signal in a deep part of the scattering medium (for example, around a two-headed arrow). In comparison with the non-focus-shift average, additional SNR gain calculated from the MFCF is 3.6 dB, which is calculated from the same region in FIG. 6.

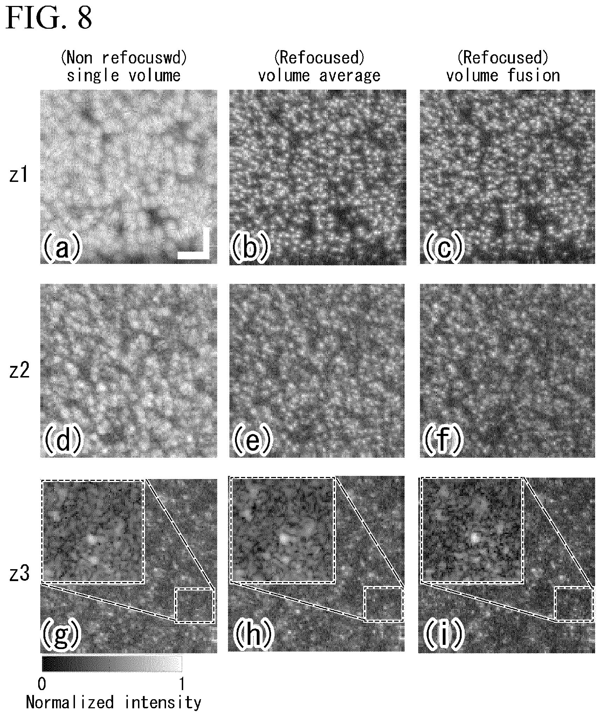

FIG. 8 is a diagram illustrating Example 4 of a processing result of the information processing device according to the modification of the embodiment. FIG. 8 depicts a frontal image of logarithm intensity in which three different depths in a phantom are selected and logarithm intensity is normalized by the maximum intensity. Referring to FIG. 8, an improvement in image quality brought by the removal of multiply scattered noise by the proposed method will be intuitively described.

In FIG. 8, images in the left column ((a), (d), (g)) are non refocused single volume images, images ((b), (e), (h)) in the middle column are images obtained by averaging six refocused non-focus-shift volumes, and images ((c), (f), (i)) in the right column are images of six refocused focus-shift volumes by the MFCF processing.

According to the images ((b), (e), (h)) in the middle column and the images ((c), (f), (i)) in the right column, it is understood that portions of the particles are sharpened by refocusing.

At a shallow depth z1, there is no noticeable difference between the non-focus-shift method and the MFCF method (see (b) and (c)). It is understood that, in deep regions z2 and z3, the MFCF image can obtain a higher contrast than the unfocused image. This indicates that the multiply scattered light is sufficiently removed.

The information processing device 10 according to the present embodiment and the information processing device 10a according to the modification of the embodiment can achieve advantageous effects similar to those described above.

The information processing device 10 according to the present embodiment and the information processing device 10a according to the modification of the embodiment can be applied to the visualization of a disease in a deep part of the fundus (choroid, sclera) and the visualization of the ciliary body of the anterior ocular segment in the ophthalmic field.

These sites have been difficult to be visualized or have been visualized only in an indistinct manner. The information processing device 10 and the information processing device 10a make it possible to visualize these sites. Therefore, it is possible to largely expand the range of diseases that can be diagnosed by ophthalmic OCT.

In the field of the OCT microscope, spheroids and organoids are becoming important measurement targets. It is possible to measure the entire depth of a spheroid with the current OCT microscope, but it is difficult to measure a deep part of a tissue in some organoids.

Furthermore, techniques for culturing spheroids and organoids are continuously improved, and it is expected that the imaging depth required for the measurement of these tissues further increases. The information processing device 10 and the information processing device 10a can meet the above requirement.

The embodiments of the present invention have been described thus far. However, the above-described embodiments are described as examples, and not intended to limit the scope of the invention. The embodiments can be implemented in a variety of other aspects, and various omissions, substitutions, changes, and combinations may be made without departing from the spirit of the invention. The embodiments and modifications thereof are included in the scope and spirit of the invention, and are also included in the invention described in the claims and the scope equivalent thereto.

Each of the information processing device 10 and the information processing device 10a described above includes a computer therein. The process of each processing of the above-described devices is stored in a computer-readable recording medium in the form of a program, and the above processing is performed by a computer reading out and executing the program. The computer-readable recording medium refers to a magnetic disk, a magneto-optical disk, a CD-ROM, a DVD-ROM, a semiconductor memory, or the like. The computer program may also be distributed to a computer via a communication line, and the computer that receives this distribution may execute the program.

The program described above may be configured to achieve some of the functions described above.

Furthermore, the functions described above may be achieved in combination with a program already recorded in the computer system, that is, the program may be a so-called differential file (differential program).


Reference Signs List

10, 10a    Information processing device

| 12 | Input unit |
| 13 | Reception unit |
| 14 | Processing unit |
| 16, 16a | Synthesis unit |
| 18 | Output unit |
| 19 | Storage unit |

**Claims**

1. An information processing device comprising:

   a reception unit configured to receive a plurality of OCT images captured under different image-capturing conditions and represented by complex signals;
   an image processing unit configured to perform digital refocusing by complex signal processing or digital aberration correction by the complex signal processing on each of the plurality of OCT images received by the reception unit; and
   a synthesis unit configured to synthesize the plurality of OCT images on which the image processing unit has performed the digital refocusing by the complex signal processing or the digital aberration correction by the complex signal processing.

2. The information processing device according to claim 1, wherein each of the plurality of OCT images is captured using an optical system having a different aberration.

3. The information processing device according to claim 2, wherein each of the plurality of OCT images is captured at a different focal position or captured using an optical system having a different defocus aberration.

4. The information processing device according to claim 1, wherein
   each of the plurality of OCT images is captured at a different focal position obtained by any of:

   changing a distance with respect to a specimen for a measuring beam, the specimen in an incident direction to an objective lens, or the objective lens;
   utilizing an optical system configured to change refractive power;
   using an electrically controlled tunable lens;
   utilizing a spatial light modulator; and
   rotating an optical element having aberration.

5. The information processing device according to claim 1, wherein each of the plurality of OCT images is captured by performing at least one of a change in a position of an objective lens, a change in a position of a specimen, or a change in an angle of the specimen with respect to an incident direction of a measuring beam to the objective lens.

6. The information processing device according to claim 1, wherein the image processing unit performs any of forward-model-based refocusing, digital adaptive optics, and interferometric synthetic aperture microscopy.

7. The information processing device according to any one of claims 1 to 5, wherein the synthesis unit performs complex averaging or intensity and amplitude averaging based on the plurality of OCT images.

8. The information processing device according to claim 6, wherein the synthesis unit corrects a relative phase between the plurality of OCT images and then synthesizes the plurality of OCT images.

9. An information processing method executed by a computer, the method comprising:

   receiving a plurality of OCT images captured under different image-capturing conditions and represented by complex signals;
   performing digital refocusing by complex signal processing or digital aberration correction by the complex signal processing on each of the plurality of OCT images; and
   synthesizing the plurality of OCT images on which the digital refocusing by the complex signal processing or the digital aberration correction by the complex signal processing has been performed.

10. A program for causing a computer to execute a process, the process comprising:

receiving a plurality of OCT images captured under different image-capturing conditions and represented by complex signals;
performing digital refocusing by complex signal processing or digital aberration correction by the complex signal processing on each of the plurality of OCT images; and

synthesizing the plurality of OCT images on which the digital refocusing by the complex signal processing or the digital aberration correction by the complex signal processing has been performed.

# FIG. 1

10

INFORMATION PROCESSING DEVICE

12
INPUT UNIT

19
STORAGE UNIT

13
RECEPTION UNIT

16
SYNTHESIS UNIT

18
OUTPUT UNIT

# FIG. 2

START

RECEIVE PLURALITY OF OCT IMAGES — S1-1

SYNTHESIZE — S2-1

OUTPUT — S3-1

END

# FIG. 3

INFORMATION PROCESSING DEVICE 10a

INPUT UNIT 12

RECEPTION UNIT 13

PROCESSING UNIT 14

SYNTHESIS UNIT 16a

STORAGE UNIT 19

OUTPUT UNIT 18

# FIG. 4

START

↓

RECEIVE PLURALITY OF OCT IMAGES — S1-2

↓

DIGITALLY REFOCUS EACH OF PLURALITY OF OCT IMAGES — S2-2

↓

SYNTHESIZE — S3-2

↓

OUTPUT — S4-2

↓

END

FIG. 5

FIG. 6

FIG. 7

EP 4 417 965 A1

# FIG. 8

(Non refocuswd) single volume    (Refocused) volume average    (Refocused) volume fusion

z1 (a) (b) (c)

z2 (d) (e) (f)

z3 (g) (h) (i)

0    1
Normalized intensity

## INTERNATIONAL SEARCH REPORT

| International application No. |
|---|
| **PCT/JP2022/038043** |

**A.    CLASSIFICATION OF SUBJECT MATTER**

*G01N 21/17*(2006.01)i
    FI:   G01N21/17 630

According to International Patent Classification (IPC) or to both national classification and IPC

**B.    FIELDS SEARCHED**

Minimum documentation searched (classification system followed by classification symbols)

    G01N 21/00-G01N 21/01, G01N 21/17-G01N 21/61, A61B 1/00-A61B 3/18

Documentation searched other than minimum documentation to the extent that such documents are included in the fields searched

    Published examined utility model applications of Japan 1922-1996
    Published unexamined utility model applications of Japan 1971-2022
    Registered utility model specifications of Japan 1996-2022
    Published registered utility model applications of Japan 1994-2022

Electronic data base consulted during the international search (name of data base and, where practicable, search terms used)

    JSTPlus/JMEDPlus/JST7580 (JDreamIII); IEEE Xplore

**C.    DOCUMENTS CONSIDERED TO BE RELEVANT**

| Category* | Citation of document, with indication, where appropriate, of the relevant passages | Relevant to claim No. |
|---|---|---|
| A | JP 2019-97944 A (NIDEK CO., LTD.) 24 June 2019 (2019-06-24)<br>    claim 1, paragraphs [0002], [0016], [0028], [0039]-[0048] | 1-10 |
| A | US 2020/0103215 A1 (CYLITE PTY LTD.) 02 April 2020 (2020-04-02)<br>    paragraphs [0109]-[0117] | 1-10 |
| A | KUMAR, Abhishek et al. Subaperture correlation based digital adaptive optics for full field optical coherence tomography. Optics Express. 06 May 2013, vol. 21, no. 9, pages 10850-10866<br>    fig. 3-8 | 6 |
| A | LI, En et al. Three-dimensional multi-contrast imaging of in vivo human skin by Jones matrix optical coherence tomography. Biomedical Optics Express. 01 May 2017, vol. 8, no. 3, pages 1290-1305<br>    fig. 1-7 | 1-10 |
| A | WANG, Lei et al. The Fusion of Multi-Focus Images Based on the Complex Shearlet Features-Motivated Generative Adversarial Network. Journal of Advanced Transportation. 30 July 2021, vol. 2021, pages 1-10<br>    fig. 1 | 1-10 |

☐ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **09 December 2022** | **20 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/038043**

| Patent document cited in search report | | | Publication date (day/month/year) | Patent family member(s) | Publication date (day/month/year) |
|---|---|---|---|---|---|
| JP | 2019-97994 | A | 24 June 2019 | (Family: none) | |
| US | 2020/0103215 | A1 | 02 April 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021169481 A

**Non-patent literature cited in the description**

- **DAWID BORYCKI ; MICHAL HAMKALO ; MACIEJ NOWAKOWSKI ; MACIEJ SZKULMOWSKI ; MACIEJ WOJTKOWSKI.** Spatiotemporal optical coherence (STOC) manipulation suppresses coherent cross-talk in full-field swept-source optical coherence tomography. *BIOMEDICAL OPTICS EXPRESS,* 01 April 2019, vol. 10 (4), 2032